(19) Europäisches Patentamt
European Patent Office
Office européen des brevets

(11) **EP 3 576 871 B1**

(12) **EUROPÄISCHE PATENTSCHRIFT**

(45) Veröffentlichungstag und Bekanntmachung des Hinweises auf die Patenterteilung:
**28.07.2021   Patentblatt 2021/30**

(21) Anmeldenummer: **18725400.8**

(22) Anmeldetag: **26.01.2018**

(51) Int Cl.:
**B01J 23/755** (2006.01)     **B01J 23/889** (2006.01)
**B01J 21/04** (2006.01)      **B01J 37/03** (2006.01)
**B01J 37/06** (2006.01)      **B01J 37/08** (2006.01)
**C07C 1/04** (2006.01)       **C07C 1/12** (2006.01)
**C10G 2/00** (2006.01)

(86) Internationale Anmeldenummer:
**PCT/EP2018/051996**

(87) Internationale Veröffentlichungsnummer:
**WO 2018/141649 (09.08.2018 Gazette 2018/32)**

(54) **MANGANDOTIERTE NICKEL-KATALYSATOREN ZUR METHANISIERUNG VON KOHLMONOXID AND KOHLDIOXID**

MANGANESE DOPED NICKEL BASED CATALYSTS FOR THE METHANATION OF CARBON MONOXIDE AND CARBON DIOXIDE

CATALYSEURS À BASE DE NICKEL DOPÉS AVEC DU MANGANÈSE POUR LA MÉTHANATION DU MONOXYDE ET DU DIOXYDE DE CARBONE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.01.2017   DE 102017000874**

(43) Veröffentlichungstag der Anmeldung:
**11.12.2019   Patentblatt 2019/50**

(73) Patentinhaber: **Clariant Produkte (Deutschland) GmbH**
**65929 Frankfurt am Main (DE)**

(72) Erfinder:
- **KOEHLER, Klaus**
  **85737 Ismaning (DE)**
- **THOMYS, Oliver**
  **85375 Neufahrn bei Freising (OT Mintraching) (DE)**
- **HINRICHSEN, Kai-Olaf**
  **85452 Eichenried (DE)**
- **KOSCHANY, Franz**
  **86415 Mering (DE)**
- **BURGER, Thomas**
  **85354 Freising (DE)**

(74) Vertreter: **Kuba, Stefan et al**
**Clariant Produkte (Deutschland) GmbH**
**IPM / Patent & License Management**
**Arabellastraße 4a**
**81925 München (DE)**

(56) Entgegenhaltungen:
**CN-A- 106 268 858**

- **SUNHWAN HWANG ET AL: "Methanation of Carbon Dioxide Over Mesoporous Nickel-M-Alumina (M = Fe, Zr, Ni, Y, and Mg) Xerogel Catalysts: Effect of Second Metal", CATALYSIS LETTERS, KLUWER ACADEMIC PUBLISHERS-PLENUM PUBLISHERS, NE, Bd. 142, Nr. 7, 18. Mai 2012 (2012-05-18), Seiten 860-868, XP035079940, ISSN: 1572-879X, DOI: 10.1007/S10562-012-0842-0**
- **SUNHWAN HWANG ET AL: "Methanation of carbon dioxide over mesoporous Ni-Fe-Al2O3 catalysts prepared by a coprecipitation method: Effect of precipitation agent", JOURNAL OF INDUSTRIAL AND ENGINEERING CHEMISTRY, Bd. 19, Nr. 6, 1. November 2013 (2013-11-01), Seiten 2016-2021, XP055480070, KOREA ISSN: 1226-086X, DOI: 10.1016/j.jiec.2013.03.015**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **ANMIN ZHAO ET AL: "Ni/Al2O3 catalysts for syngas methanation: Effect of Mn promoter", JOURNAL OF NATURAL GAS CHEMISTRY., Bd. 21, Nr. 2, 1. März 2012 (2012-03-01), Seiten 170-177, XP055480082, US, CN ISSN: 1003-9953, DOI: 10.1016/S1003-9953(11)60350-2**
- **WAN AZELEE WAN ABU BAKAR ET AL: "Nickel Oxide Based Supported Catalysts for the In-situ Reactions of Methanation and Desulfurization in the Removal of Sour Gases from Simulated Natural Gas", CATALYSIS LETTERS, KLUWER ACADEMIC PUBLISHERS-PLENUM PUBLISHERS, NE, Bd. 128, Nr. 1-2, 11. November 2008 (2008-11-11), Seiten 127-136, XP019671959, ISSN: 1572-879X**
- **ZHAO KECHAO ET AL: "CO2methanation and co-methanation of CO and CO2over Mn-promoted Ni/Al2O3catalysts", FRONTIERS OF CHEMICAL SCIENCE AND ENGINEERING, HIGHER EDUCATION PRESS, HEIDELBERG, Bd. 10, Nr. 2, 14. März 2016 (2016-03-14), Seiten 273-280, XP035946675, ISSN: 2095-0179, DOI: 10.1007/S11705-016-1563-5 [gefunden am 2016-03-14]**

**Beschreibung**

Die Energieversorgung durch die sogenannten erneuerbaren Energien Photovoltaik und Windenergie leidet unter der Problematik der witterungs- und tageszeitabhängigen Schwankungen in der Stromproduktion. Zur Gewährleistung der Versorgungssicherheit muss ein Weg gefunden werden, um witterungs- und tageszeitabhängige Schwankungen in der Stromproduktion abzufangen. Eine potentielle Methode, um die Energie chemisch zu speichern, stellt das Power-to-Gas-Verfahren dar, in welchem überschüssiger Strom verwendet wird, um Wasser durch Elektrolyse in Wasserstoff und Sauerstoff zu spalten. Wasserstoff, in welchem die Energie nach der Wasserelektrolyse gespeichert ist, kann selbst nur mit hohem Aufwand gelagert oder zum Verbraucher transportiert werden. Nach dem Power-to-Gas-Verfahren wird der Wasserstoff daher in einem weiteren Schritt mit Kohlendioxid, welches in der Atmosphäre als klimaschädliches Treibhausgas wirkt, in der Methanisierungsreaktion zu Methan und Wasser umgesetzt. Methan kann in bereits vorhandenen Infrastrukturen, welche Kapazitäten zur Speicherung im Bereich von mehreren Monaten umfassen, leicht gespeichert, über größere Strecken nahezu verlustfrei transportiert und in Zeiten von Energiebedarf wieder rückverstromt werden. Die Methanisierungsreaktion, die mit einer hohen Energiefreisetzung verbunden ist und üblicherweise katalysiert abläuft, bildet das Kernstück des Verfahrens. Aus der hohen Exothermie der Reaktion (-165 kJ/mol) ergeben sich zwei direkte Probleme. Zum einen limitiert bei hohen Temperaturen das thermodynamische Gleichgewicht die maximal erreichbare Ausbeute an Methan. Für die Einspeisung von Methan in das Erdgasnetz ist in Deutschland eine Reinheit von 95% notwendig. Daraus ergibt sich die Forderung nach einer hohen Katalysatoraktivität, sodass bei industriell angewandten Reaktionsdrücken bei niedrigen Temperaturen höhere Ausbeuten an Methan erzielt werden können.

Die hohe Exothermie der Methanisierungsreaktion bewirkt bei adiabatischer Reaktorfahrweise unter Umständen Hotspotbildungen im Reaktor. Dabei kommt es lokal zur Entwicklung hoher Temperaturen, die den Katalysator beschädigen können. Es besteht daher ein starkes Interesse darin, möglichst stabile Katalysatorsysteme zu entwickeln, um Kosten für Katalysatorwechsel/Stillstandzeit und Materialkosten zu minimieren. Weiterhin ergibt sich der Bedarf von möglichst aktiven und selektiven Katalysatoren für die Methanisierungsreaktion von $CO_2$.

Für die Methanisierungsreaktion werden hauptsächlich nickelbasierte Katalysatoren verwendet, jedoch gibt es auch Ansätze mit anderen aktiven Metallen, wie beispielsweise Ruthenium oder Rhodium. Als Träger kommen neben Aluminiumoxid auch die Oxide von Silizium, Titan und Zirkonium zum Einsatz. Beschrieben werden solche Systeme zum Beispiel in den Offenlegungsschriften CN 104043454 A, CN 103480375 A, CN 102091629 A, CN 104888783 A und WO 2008110331 A1.

Zusätzlich findet man Katalysatoren, welche nicht nur ein aktives Metall enthalten, sondern promotiert sind. Aus der Literatur ist bekannt, dass eine Promotierung mit Eisen oder Mangan einen positiven Einfluss auf die Katalysatorperformance haben kann.

Eine Dotierung mit Mangan wird zum Beispiel in CN 103464163 A, CN 103752315 A, CN 102600860 A, CN 102553610 A, CN 102527405 A, CN 102513124 A, CN 102463120 A, CN 102463119 A, CN 103706366, CN 106858268 A, CN 104028270 und CN 101745401 beschrieben sowie durch Zhao et al., Journal of Natural Gas Chemistry (2012), 21(2), 170-177, Wan Azelee Wan Abu Bakar et Al, Catalysis Letters (2008), 128 (1-2), 127-136, Zhao Kechao et Al, Frontiers of Chemical Science and Engineering, (2016) 10(2), 273-280, und Gao et al. Journal of molecular catalysis (China), Vol. 25, No. 1, Feb. 2011.

CN 103752315 A betrifft einen auf einer metallischen Trägerphase vorliegenden Katalysator sowie das Herstellungsverfahren und die Anwendung des Katalysators in der Herstellung von Methan bzw. der synthetischen Methangasherstellung durch die katalytische Umwandlung von Kohlenmonoxid und/oder Kohlendioxid. Der auf der metallischen Trägerphase vorliegende Katalysator umfasst als aktive Komponente ein Metalloxid, das auf der metallischen Trägerphase vorliegt und ein Hilfsmetalloxid, die allgemeine Formel des auf der metallischen Trägerphase vorliegenden Katalysators ist die folgende: xM1O-yM2O/ZT, wobei M1O das Metalloxid als aktive Komponente ZT die metallische Trägerphase, x den gewichtsprozentualen Anteil des Metalloxids als aktiven Komponente im Katalysator und y den gewichtsprozentualen Anteil des Hilfsmetalloxids darstellt.

CN 102600860 A betrifft einen Katalysator, der zur vollständigen Methanisierung von synthetischen Gas mittlerer oder tieferer Temperaturen geeignet ist. Der Katalysator umfasst die folgenden Komponenten in Gewichtsprozent: 12 bis 26 % Nickel, 1 bis 4 % Mangan, 0,1 bis 2 % einer oder mehrerer aus Lanthan, Calcium und/oder Magnesium und im Übrigen einen Kompositträger, enthaltend Aluminiumoxid und Ceroxid, wobei die Summe der Gewichtsprozente der Komponenten 100 % ist, und der Ceroxid-Gehalt in den Kompositträger 6 bis 12 % ist. Der Katalysator wird hergestellt durch die Schritte: (1) Bilden des Kompositträgers, (2) Herstellen der Imprägnierlösung; (3) Behandeln durch Anpassen der Druck-Imprägnations-Methode; und (4) Erhitzen des imprägnierten Katalysators auf 500 bis 600 °C; und Kalzinieren für 6 bis 8 Stunden, um das gewünschte Produkt zu erhalten.

CN 102527405 A offenbart einen Katalysator der zur vollständigen Methanisierung von Synthesegas bei hoher Temperatur verwendet wird, wobei der Katalysator die folgenden Bestandteile in Gewichtsprozent umfasst: 10 bis 30 Gew.-% Nickel, 11 bis 20 Gew.-% Lanthan, 1 bis 5 Gew.-% Cer, 0,1 bis 2 Gew.-% einer oder mehrerer aus Mangan, Lithium und Vanadium und im Übrigen Aluminiumoxid, wobei die Summe der Gewichtsprozente all dieser Bestandteile 100 % ist. Das Herstellungsverfahren des Katalysators umfasst die folgenden Schritte: (1) Kombinieren und Vermischen der Katalysator-Bestandteile; (2) Trocknen um ein Gemisch zu erhalten; (3) Erhitzen des Gemisches auf 600 bis 800 °C mit einer Aufheiz-Geschwindigkeit von 1 bis 4 °C pro Minute und Kalzinieren bei einer Temperatur zwischen 600 bis 800 °C für 6 bis 8 Stunden, um ein pulverförmiges Produkt zu erhalten, und (4) Vermahlen des pulverförmigen Produktes und Behandeln mit einem Druck-Imprägnations-Verfahren, um das Zielprodukt zu erhalten.

CN 102513124 A offenbart eine Technologie zur Herstellung von künstlichem Erdgas durch Methanisierung von Kohlenstoff-Oxiden und stellt einen Katalysator zur Methanisierung von Koksofengas sowie eine Herstellungsmethode desselben zur Verfügung. Der Katalysator ist ein $\gamma$-Aluminiumoxid oder ein Titandioxid-Träger, um die aktive Komponente und ein Zusatzmittel zu stabilisieren, wobei die aktive Komponente aus Ni und das Zusatzmittel aus einem 1. Zusatzmittel und einem 2. Zusatzmittel besteht; das 1. Zusatzmittel ist ein Seltenerdelement und das 2. Zusatzmittel ist eines oder eine Kombination mehrerer aus Sr, Mn, V, Zr, Ce und Cr.

CN 102463120 A offenbart einen Katalysator, der Nickel und Mangan enthält, seine Herstellung und seine Verwendung. Der Katalysator, der Nickel und Mangan enthält, enthält a) Nickeloxid, wobei der Nickelgehalt im Bereich von 15 bis 55 Gew.-% ist und b) Manganoxid wobei der Mangangehalt im Bereich von 0,1 bis 15 Gew.-% liegt. Mindestens eines aus Aluminiumoxid, Siliziumoxid, Titanoxid und Zirkonoxid wird als Katalysatorträger verwendet. Das Herstellungsverfahren umfasst die Schritte des Lösens eines Nickelsalzes und eines Mangansalzes in einer organischen sauren Lösung, Benetzen des Katalysatorträgers mit der gemischten Lösung, die im vorangegangenen Schritt erhalten wurde, Trocknen in einer Thermolyse, um den Nickel- und Mangan-enthaltenden Katalysator zu erhalten. Die organische Säurelösung ist eine organische Carboxyl-Säure oder eine reduzierende organische Säure, wobei das Molverhältnis der Gesamtbeladung der Carboxylionen der organischen Säure zu den Metallionen zwischen 0,5 bis 3:1 ist und das Molverhältnis der reduzierenden organischen Säure zu den Metallionen 0,2 bis 2:1 ist.

CN 102463119 A offenbart einen Methanisierungskatalysator und die Herstellung desselben. Der Methanisierungskatalysator umfasst die folgenden, Komponenten: a) Nickeloxid, wobei der Nickel-Gehalt zwischen 5 bis 50 Gew.-% liegt; b) Manganoxid, wobei der Mangan-Gehalt von 0,1 bis 15 Gew.-% liegt; c) mindestens eines der Oxide von Beryllium, Mangan, Calcium, Strontium, Barium, Lanthan und Cer, wobei der Metallgehalt zwischen 0,5 bis 20 Gew.-% liegt; die Komponente c) verteilt im gesamten Trägerpartikel vorliegt. Die offenbarte Herstellung umfasst die folgenden Schritte: 1) Gemeinsames Lösen eines Nickelsalzes und/oder eines Mangansalzes mit einer organischen Säure, Herstellen eines Salzes der Komponente c) in einer wässrigen Lösung; und 2) Benetzen der zu stabilisierenden Komponenten, des Nickels, des Mangans und der Komponente c), wobei die Komponente c) unabhängig stabilisiert wird und Kalzinieren sowie eine Sinter-/Zersetzungsbehandlung nach jedem Stabilisieren durchgeführt wird.

CN 103706366 beschreibt einen Katalysator umfassend 15 bis 55 % einer aktiven Komponente, 1 bis 6 % eines 1. katalytischen Promotors, 3 bis 15 % eines 2. katalytischen Promotors und im Übrigen einen katalytischen Träger, wobei die aktive Komponente Nickeloxid ist, der katalytische Träger $Al_2O_3$ ist, der 1. katalytische Promotor einer ist ausgewählt aus Lanthanoxid, Ceroxid oder Samariumoxid, der 2. katalytische Promotor mindestens einer ist aus Magnesiumoxid, Manganoxid, Eisenoxid und Zirkonoxid. Die Herstellungsmethode umfasst die Schritte: Auflösen eines Reduktionsmittels in Wasser, um eine reduzierende Lösung zu erhalten, Einmischen von Nickelnitrat, Aluminiumnitrat, dem 1. Metallsalz und dem 2. Metallsalz in Wasser, um eine Rohmateriallösung zu erhalten und Hinzugeben der reduzierenden Mischung zur Rohmateriallösung unter gleichmäßigen Rühren, um eine 1. Lösung zu erhalten, Übertragen in einen abgedichteten Reaktor und Durchführen einer Reaktion bei 100 bis 200 °C für 10 bis 15 Stunden, Kühlen auf Raumtemperatur, Filtrieren und Waschen bis zu einem pH-Wert von 6 bis 7, Trocknen bei 80 bis 120 °C für 4 bis 30 Stunden und Kalzinieren bei 300 bis 550 °C für 2 bis 12 Stunden, um einen Katalysator zu erhalten, in dem das Reduktionsmittel eines ist ausgewählt aus Formaldehyd, HydrazinHydrat, Natriumhypophosphit und Ascorbinsäure, wobei das 1. Metallsalz eines ist ausgewählt aus Lanthannitrat, Cernitrat und Samariumnitrat, das 2. Metallsalz mindestens eines ist ausgewählt aus Magnesiumnitrat, Manganitrat, Eisennitrat und Zirkonnitrat, die AlkaliLösung eine wässrige Lösung aus Natriumcarbonat, Natriumhydroxid, Kaliumcarbonat, Ammoniumcarbonat, Harnstoff oder $NH_3*H_2O$ ist. Der Katalysator kann mit einem Bindemittel (Calciumaluminat), einem Schmiermittel (Graphit) und Wasser vermischt werden und dann in Partikelform gepresst werden. Der Katalysator ist zur Methanisierung von Kohlegas bei hoher Temperatur und hohem Druck geeignet, um synthetisches Erdgas zu produzieren.

CN 104028270 offenbart einen Methanisierungskatalysator umfassend 5 bis 60 Gew.-% einer katalytisch aktiven NiO-Komponente, basierend auf dem Gesamtgewicht des Katalysators und im Übrigen $Al_2O_3$, der auch 1 bis 25 Gew.-%, basierend auf dem Gesamtgewicht des Katalysators, an mitwirkender Komponente M umfassen kann, wobei die mit-wirkende Komponente M ausgewählt ist aus einem oder mehreren Oxiden der Metalle Ce, Ca, Co, La, Sm, Zr, Ba, Mn, Fe, Mo, Ti und Cu. Das Dokument stellt auch ein Verfahren zur Herstellung des Methanisierungskatalysators zur Verfügung, welches das Mischen der Vorstufe der katalytisch aktiven Komponente, der Vorstufe der mitwirkenden Komponente M, sowie eines Katalysatorträgers - gemäß dem entsprechenden Anteil in der Methanisierungskatalysator-Zusammensetzung - das Hinzugeben eines organischen Brennstoffes und das gründliche Vermischen und Trocknen, um ein gelartiges Produkt zu formen, das Durchführen einer Verbrennungsreaktion, das Säubern und das Trocknen, um das endgültige Produkt zu erhalten, beinhaltet.

CN 101745401 offenbart einen geträgerten schwefelresistenten Methanisierungskatalysator, welcher sich dadurch auszeichnet, dass er ein Hauptmetall M als aktive Komponente ein 2. Metall M1 als Hilfsstoff und S als Trägermaterial beinhaltet, wobei das Gewichtsverhältnis zwischen M1, M und S zwischen 0,01 bis 39 : 1 bis 30 : 0,01 bis 90 ist, M Mo, W und/oder V ist, das 2. Metall M1 eines oder mehrere aus Fe, Co, Ni, Cr, Mn, La, Y oder/und Ce ist und der Träger S $ZrO_2$, $Al_2O_3$, MgO oder $TiO_2$ ist. Der geträgerte schwefelresistente Methanisierungskatalysator wird durch eine Sol-Gel-Methode dargestellt.

Es ist Aufgabe der Erfindung einen Methanisierungskatalysator zur Methanisierung von Kohlenmonoxid und/oder Kohlendioxid bereitzustellen, der bei hoher Selektivität und Stabilität eine gegenüber den Katalysatoren des Stands der Technik verbesserte Aktivität aufweist.

Diese Aufgabe wird gelöst durch einen Katalysator zur Methanisierung von Kohlenmonoxid und/oder Kohlendioxid, umfassend Aluminiumoxid, eine Ni-Aktivmasse sowie Mn, dadurch gekennzeichnet, dass das molare Ni/Mn-Verhältnis im Katalysator von 4,0 bis 6.5 beträgt und das atomare Al/Ni-Verhältnis zwischen 0,5 und 1.5 ist.

Bei dem Aluminiumoxid muss es sich nicht um stöchiometrisches $Al_2O_3$ handeln, vielmehr kann es sich hierbei auch um ein nicht-stöchiometrisches Aluminiumoxid handeln.

Der Promotor Mn kann vollständig oder teilweise in der Ni-Aktivmasse enthalten sein. Der Katalysator kann neben Mn weitere Promotoren enthalten, er kann aber auch ausschließlich den Promotor Mn enthalten. Die Oxidationsstufen von Al, Ni sowie der Promotoren können je nach Behandlung des Katalysators variieren. Al, Ni und die Promotoren liegen typischerweise als Metallkationen (z.B. $Al^{3+}$, $Ni^{2+}$, $Mn^{2+}$, $Mn^{3+}$, $Mn^{4+}$) vor. Nach der Kalzinierung, z.B. an Luft, können hohe Oxidationsstufen bzw. die maximalen Oxidationsstufen erreicht werden. Wird der Katalysator bei Temperaturen oberhalb Raumtemperatur reduziert, z.B. unter Reaktionsbedingungen mit Wasserstoff, können Al, Ni und die Promotoren niedrigere Oxidationsstufen annehmen oder teilweise oder ganz in der Oxidationsstufe 0 auftreten. Der Ladungsausgleich zu den Metallkationen erfolgt durch Sauerstoffanionen ($O^{2-}$). Der erfindungsgemäße Katalysator kann weitere Komponenten, neben Aluminiumoxid ($AlO_x$ mit $x \leq 1,5$) Ni, und Mn (sowie die zum Ladungsausgleich notwendigen Sauerstoffanionen) enthalten, er kann jedoch auch ausschließlich aus Aluminiumoxid, Ni und Mn bestehen. Auch kann die Ni-Aktivmasse neben Mn weitere Promotoren enthalten, darunter zum Beispiel Fe, sie kann aber auch ausschließlich den Promotor Mn enthalten. Vorzugsweise enthält die Ni-Aktivmasse keines der Elemente ausgewählt aus Ta, In, Cu, Ce, Cr, Bi, Fe, P, Sb, Sn, B, Si, Ti, Zr, Co, Rh, Ru, Ag, Ir, Pd und Pt. Vorzugsweise enthält die Ni-Aktivmasse kein Edelmetall.

Das atomare Al/Ni-Verhältnis ist zwischen 0,5 und 1,5 sein, bevorzugterweise zwischen 0,8 und 1,2, besonders bevorzugt ist das Al/Ni-Verhältnis annähernd 1.

Die erfindungsgemäßen Katalysatoren können vorteilhafterweise in der Ni-Aktivmasse Kristallite mit einem Durchmesser unter 20 nm, bevorzugt unter 10 nm, aufweisen. Die Ni-Aktivmasse kann auch ganz oder zu wesentlichen Teilen aus Kristalliten mit einem Durchmesser unter 20 nm, bevorzugt unter 10 nm bestehen. Die Ni-Aktivmasse liegt vorzugsweise in einem metallischen Zustand vor.

Die $CO_2$-Aufnahmekapazität der Katalysatoren bei 35 °C kann größer 150 $\mu$mol/g sein und liegt vorzugsweise im Bereich zwischen 150 bis 350 $\mu$mol/g, besonders bevorzugt zwischen 180 bis 260 $\mu$mol/g.

Die BET-Oberfläche ($S_{BET}$) des erfindungsgemäßen Katalysators kann größer als 100 $m^2$/g sein, vorzugsweise größer als 200 $m^2$/g, oder im Bereich zwischen 200 und 400 $m^2$/g, vorzugsweise im Bereich zwischen 200 und 300 $m^2$/g und insbesondere im Bereich zwischen 200 und 250 $m^2$/g.

Die spezifische Metalloberfläche ($S_{Met}$) des erfindungsgemäßen Katalysators ist vorzugsweise größer als 5 $m^2$/g, vorzugsweise größer als 10 $m^2$/g oder im Bereich zwischen 10 und 30 $m^2$/g, vorzugsweise im Bereich zwischen 15 und 25 $m^2$/g oder im Bereich zwischen 17 und 23 $m^2$/g.

Weiterhin betrifft die Erfindung ein Verfahren zur Herstellung eines Methanisierungskatalysators, umfassend die Schritte:

a) Kopräzipitation aus einer Lösung, die Al, Ni und Mn in gelöster Form enthält, um einen Niederschlag zu erhalten,
b) Isolieren des Niederschlags aus Schritt a),
c) Trocknen des isolierten Niederschlags aus Schritt b) und
d) Kalzinieren des getrockneten Niederschlags aus Schritt c).

Bevorzugt ist hierbei, dass die Lösung aus Schritt a) eine wässrige Lösung ist und Al, Ni und Mn in der wässrigen Lösung gelöst als ionische Verbindungen vorliegen.

Al liegt bevorzugterweise gelöst als Aluminiumnitrat, Aluminiumtrichlorid oder Aluminiumsulfat vor. Ni liegt bevorzugterweise gelöst als Nickelnitrat, Nickeldichlorid, Nickelsulfat, Nickelacetat oder Nickelcarbonat vor. Mn liegt bevorzugterweise in der Oxidationsstufe II oder IV vor und ist als Mangannitrat, Manganacetat, Mangandichlorid, Mangansulfat oder auch in der Oxidationsstufe VII als Permanganat-Ion gelöst.

Die Kopräzipitation erfolgt durch Hinzugeben der Lösung, die Al, Ni und Mn enthält, zu einer vorgelegten basischen Lösung oder durch Zugabe einer basischen Lösung zu der vorgelegten Lösung, die Al, Ni und Mn enthält.

Alternativ wird die Lösung, die Al, Ni und Mn enthält und die basische Lösung gleichzeitig in ein Gefäß gegeben, welches bereits ein Lösungsmittel wie Wasser enthalten kann und darin vermischt. Die basische Lösung weist einen pH größer 7, vorzugsweise im Bereich von 8 bis 10 auf und enthält bevorzugterweise ein Alkali-Hydroxid und/oder ein Alkali-Carbonat, zum Beispiel eine Mischung aus Natriumhydroxid und Natriumcarbonat. Die Kopräzipitation erfolgt vorzugsweise unter Temperierung, sodass die Temperatur der Lösung annähernd Raumtemperatur oder zum Beispiel 30 °C beträgt.

Bevorzugterweise liegen Al, Ni und Mn in gelöster Form, als ionische Verbindungen in der wässrigen Lösung vor und weisen dasselbe Anion auf, welches zum Beispiel Nitrat sein kann.

Nach der Fällung durch die Kopräzipitation wird vorzugsweise der Niederschlag in der Lösung für mindestens 30 Minuten, bevorzugterweise länger als 1 Stunde, stärker bevorzugt länger als 12 Stunden gealtert. Die Alterung erfolgt vorzugsweise dadurch, dass der Niederschlag bei annähernd Raumtemperatur in der Lösung (Mutterlauge) unter Rühren belassen wird.

Der durch die Kopräzipitation erhaltene Niederschlag wird isoliert, zum Beispiel indem eine Filterung durchgeführt wird. Die Filterung kann in geeigneter Weise zum Beispiel durch eine Filterpresse erfolgen.

Der isolierte Niederschlag wird vorzugsweise gewaschen, zum Beispiel mit destilliertem Wasser, bis ein neutraler pH-Wert erreicht wird.

Im Folgenden kann der isolierte Niederschlag getrocknet werden, zum Beispiel bei erhöhter Temperatur an Luft. Bevorzugterweise erfolgt die Trocknung bei einer Temperatur zwischen 70 °C und 90 °C für einen Zeitraum länger als 4 Stunden, vorzugsweise länger als 12 Stunden.

Der isolierte Niederschlag wird kalziniert, dies kann an Luft bei einer Temperatur zwischen 300 °C bis 600 °C erfolgen, bevorzugterweise bei 400 bis 500 °C und in einem Zeitraum von 3 Stunde bis 10 Stunden, bevorzugterweise 5 bis 7 Stunden.

Der erfindungsgemäße Katalysator soll insbesondere in der Methanisierung von Kohlenmonoxid und/oder Kohlendioxid Verwendung finden. Die Methanisierung von Kohlendioxid lässt sich durch die folgende Reaktionsgleichung darstellen:

$$4H_2 + CO_2 \rightarrow CH_4 + 2H_2O$$

Die Methanisierung von Kohlenmonoxid lässt sich durch die folgende Reaktionsgleichung darstellen:

$$3H_2 + CO \rightarrow CH_4 + H_2O$$

Bei dem Verfahren zur Durchführung der Methanisierung wird das Reaktionsgas, welches Kohlendioxid und/oder Kohlenmonoxid oder ein Gemisch aus beidem enthält, bei einer Temperatur oberhalb von 200 °C mit dem Katalysator in Kontakt gebracht.

Die Figuren 1 bis 4 zeigen das katalytische Verhalten der mangandotierten Katalysatoren Mn1, Mn4, Mn6 und Mn8 vor und nach Alterung.

Figur 1: Katalytische Testergebnisse zu Mn1 (Vergleichsbeispiel).

Figur 2: Katalytische Testergebnisse zu Mn4 (Beispiel).

Figur 3: Katalytische Testergebnisse zu Mn6 (Beispiel).

Figur 4: Katalytische Testergebnisse zu Mn8 (Vergleichsbeispiel).

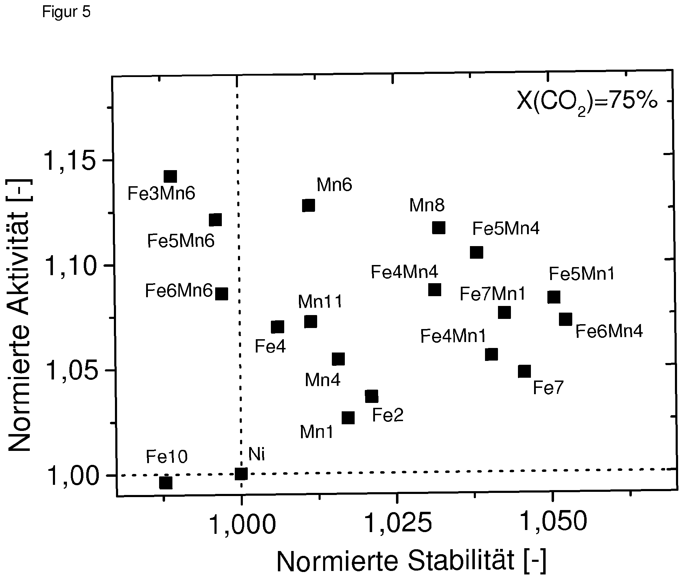

Figur 5: Aktivitäts-/Stabilitätsdiagramm der beschriebenen Proben.

**Methoden**

*Elementaranalyse*

Die Bestimmung der Zusammensetzung der kalzinierten Katalysatoren erfolgte durch optische Emissionsspektroskopie mittels induktiv gekoppeltem Plasma (ICP-OES). 50 mg an Katalysator wurden in 50 ml 1 molarer Phosphorsäure (VWR, p.A.) bei 60°C gelöst. Um gebildeten Braunstein zu lösen, wurden zur Lösung 50 mg $Na_2SO_3$ (Sigma Aldrich, p.A.) zugegeben. Nach Abkühlung wurden die Lösungen 1/10 verdünnt und mittels 0,1 $\mu$m Filtern (Pall) gefiltert. Die Kalibrierlösungen wurden zu 1, 10 und 50 mg $l^{-1}$ (Merck) angesetzt. Die Bestimmung der Metallkonzentrationen wurde mittels eines Agilent 700 ICP-OES durchgeführt.

*Bestimmung der spezifischen Oberfläche*

Die Bestimmung der spezifischen Oberflächen der Katalysatoren ($S_{BET}$) wurde mittels N2-BET Analyse an einer NOVA 4000e (Quantachrome) durchgeführt. Hierzu wurden 100 mg Katalysator für 3 Stunden bei 120°C entgast und anschließend Adsorptions- und Desorptionsisotherme im $p/p_0$-Bereich von 0,007 bis 1 aufgenommen. Zur Bestimmung der BET-Oberfläche wurden die Datenpunkte im $p/p_0$-Bereich von 0,007 bis 0,28 herangezogen.

*Chemisorption*

Chemisorptionsexperimente wurden an einer Autosorb 1C (Quantachrome) vorgenommen. Vor der Messung wurden 100 mg Katalysator bei 500 °C in 10% H2 in N2 für 6 Stunden aktiviert. Die Heizrampe betrug 2 Kmin$^{-1}$.

Die Bestimmung der Metalloberfläche ($S_{MET}$) erfolgte nach DIN 66136-2 (Ver. 2007-01) und wurde mittels H2-Chemisorption bei 35 °C durchgeführt. Hierfür wurden 20 Adsorptionspunkte äquidistant von 40 mmHg bis 800 mmHg aufgenommen. Die Equilibrierzeit für die Adsorption betrug 2 min, die des thermischen Gleichgewichts 10 min. Zur Bestimmung der Metalloberfläche wurde eine Metallatom/H-Stöchiometrie von 1 angesetzt. Für die $CO_2$-Chemisorptionsmessungen zur Bestimmung der $CO_2$-Aufnahmekapazitäten ($U(CO_2)$) wurde bei ansonsten unveränderten Parametern die Equilibrierzeit für die Adsorption auf 10 min gesetzt. Vor Aufnahme der Chemisorptionsdaten wurde eine etwaige kinetische Hemmung der $CO_2$-Chemisorption unter diesen Bedingungen experimentell ausgeschlossen. Metalloberflächen und $CO_2$-Aufnahmekapazitäten wurden gemäß der Extrapolationsmethode auf einen Druck von 0 mmHg extrapoliert.

*Synthese*

Die Katalysatoren wurden durch Kopräzipitation hergestellt und das atomare Verhältnis von Nickel und Aluminium auf 1 eingestellt. Zur Untersuchung der Wirkung von Eisen auf das Katalysatorverhalten wurde während der Katalysatorsynthese Eisen(III)-nitrat zur Salzlösung aus Nickel- und Aluminiumnitrat zugegeben. Zur Untersuchung der gleichzeitigen Wirkung von Eisen und Mangan auf das Katalysatorverhalten wurde während der Katalysatorsynthese Mangan(II)-nitrat und Eisen(III)-nitrat zur Salzlösung aus Nickel- und Aluminiumnitrat zugegeben. Die Reinheit aller verwendeter Chemikalien war p.a. Wasser wurde durch ein Millipore-Filtersystem aufgereinigt und der Reinheitsgrad mittels Leitfähigkeitsmessungen verifiziert. Die Synthese erfolgte in einem doppelwandigen, 3 l fassenden Rührkessel. Der mit Wasser gefüllte Doppelmantel ermöglichte über einen Thermostaten die Temperierung des Syntheseansatzes auf 30 °C, zwei Strömungsbrecher sorgten für eine verbesserte Vermischung. Zum Rühren kam ein KPG-Rührer mit 150 Umdrehungen min$^{-1}$ zum Einsatz. Für die Synthese wurde im Rührkessel 1 l $H_2O$ vorgelegt und auf pH = $9 \pm 0,1$ eingestellt. Die Dosierung der Mischung der gelösten Nitrate erfolgte mit 2,5 ml min$^{-1}$. Zeitgleich diente die kontrollierte Zugabe des Fällungsreagenz zur Aufrechterhaltung des pH-Werts. Als Ausgangsstoffe kamen einmolare Lösungen der jeweiligen Nitrate zum Einsatz ($Ni(NO_3)_2 * 6H_2O$, $Al(NO_3)_2 * 9H_2O$, $Fe(NO_3)_3 * 9H_2O$ und $Mn(NO_3)_2 * 4H_2O$). Diese wurden, wie in Tabelle 2 dargestellt, zu einem Gesamtvolumen von 120 ml min$^{-1}$ gemischt, bevor das Zutropfen in den Reaktor erfolgte. Als Fällungsreagenz diente eine volumengleiche Mischung aus den Lösungen 0.5M NaOH und 0.5 M $Na_2CO_3$, zu deren Dosierung ein Titrator verwendet wurde. Unter ständigem Rühren wurde die Suspension über Nacht in der Mutterlauge gealtert, der Niederschlag anschließend abfiltriert und so lange mit $H_2O$ gewaschen, bis das Filtrat einen neutralen pH-Wert aufwies. Nach einer Trocknung bei 80 °C im Trockenschrank über Nacht wurde der getrocknete Niederschlag (Prekursor) mit einer Aufheizrate von 5 K min$^{-1}$ auf 450 °C erhitzt und für 6 Stunden unter synthetischer Luft kalziniert.

*Aktivitäts- und Stabilitätsmessung*

Um verschiedene Katalysatoren in ihrer Aktivität bezüglich der $CO_2$-Methanisierung vergleichen zu können, wurde ein Testprogramm entwickelt, welches eine Aussage zu deren Aktivität und Stabilität. Dazu wurden 25 mg Katalysator in der Siebfraktion 150 - 200 μm mit der neunfachen Menge an SiC verdünnt und in den Reaktor eingebaut. Die nacheinander in der Reihenfolge Reduktion, Einlaufen, S-Kurve 1, Alterung und S-Kurve 2 durchgeführten Messschritte, sind im Detail in Tabelle 1 aufgeführt.

Tabelle 1: Parameter der Messschritte zur Ermittlung des Aktivitäts- und Stabilitätsprofils

| | Reaktionsgas, Verhältnis $H_2/CO_2/Ar$ | Reaktionsgas Q [Nl $g_{Kat}^{-1}$ h$^{-1}$] | T [°C] | $p_{abs}$ [bar] | Dauer [h] |
|---|---|---|---|---|---|
| Reduktion | 5/0/95 | 130 | 485 | 1 | 8 |
| Einlaufen | 4/1/5 | 150 | 260 | 7 | 24 |

(fortgesetzt)

| | Reaktionsgas, Verhältnis $H_2/CO_2/Ar$ | Reaktionsgas Q [Nl $g_{Kat}^{-1}$ $h^{-1}$] | T [°C] | $p_{abs}$ [bar] | Dauer [h] |
|---|---|---|---|---|---|
| S-Kurve 1 | 4/1/5 | 150 | 170 - 500 | 8 | - |
| Alterung | 4/1/5 | 150 | 500 | 7 | 32 |
| S-Kurve 2 | 4/1/5 | 150 | 170 - 500 | 8 | - |

Zur Bestimmung der Temperatur-$CO_2$-Umsatz-Kurven, wurde die Temperatur im angegebenen Bereich schrittweise um 25 °C erhöht und jeweils die Aktivität bestimmt. Ein Vergleich der beiden S-Kurven vor und nach 32 Stunden Alterung bei 500 °C lässt einen Einblick auf die Stabilität der Systeme hinsichtlich hoher Temperaturen zu.

Als Maß für die Aktivität wurde repräsentativ die Temperatur $T_{75,1}$ bestimmt, welche nötig ist, um während des Messschrittes S-Kurve 1 einen $CO_2$-Umsatz von 75% zu erreichen. Hierzu wurde die Temperatur im angegebenen Bereich schrittweise um 25 °C erhöht. Je niedriger $T_{75,1}$ ist, desto höher ist daher die Aktivität des Katalysators.

Als Maß für die Aktivität nach der Alterung wurde repräsentativ die Temperatur $T_{75,2}$ bestimmt, welche nötig ist, um während des Messschrittes S-Kurve 2 einen $CO_2$-Umsatz von 75% zu erreichen. Hierzu wurde die Temperatur im angegebenen Bereich schrittweise um 25 °C erhöht. Je niedriger $T_{75,2}$ ist, desto höher ist daher die Aktivität des Katalysators nach der Alterung.

Berechnet man die Differenz der $T_{75,1}$ und $T_{75,2}$ aus den beiden Umsatz-Temperatur-Charakteristika, so erhält man ein Maß für die Stabilität des Katalysators. Auch hier gilt, je niedriger die Differenz, desto stabiler der Katalysator. Zur besseren Vergleichbarkeit wurden alle bestimmten Aktivitäten und Stabilitäten auf die des nicht promotierten Nickel-Aluminiumoxid-Katalysators (Ni) normiert. Die normierte Aktivität und Stabilität ergibt sich aus:

$$\text{Normierte Aktivität} = \frac{T_{75,1}(Ni/AlO_x)}{T_{75,1}(dot.\ Kat.)}$$

$$\text{Normierte Stabilität} = \frac{\frac{T_{75,2}(Ni/AlO_x)}{T_{75,1}(Ni/AlO_x)}}{\frac{T_{75,2}(dot.\ Kat.)}{T_{75,1}(dot.\ Kat)}}$$

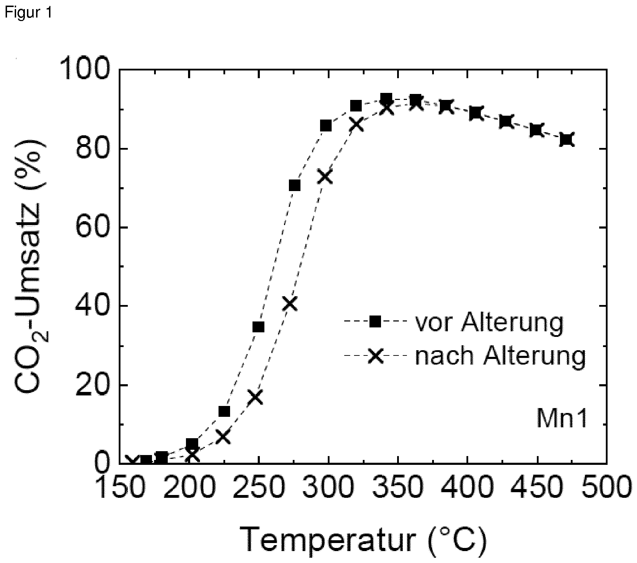

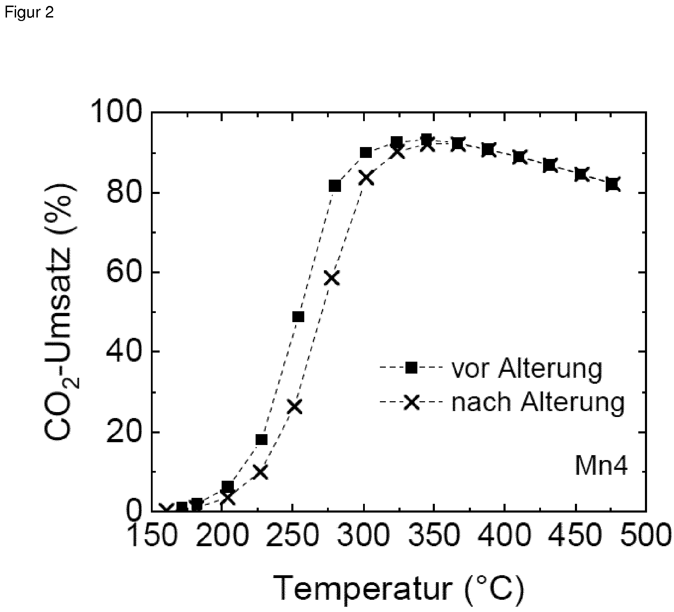

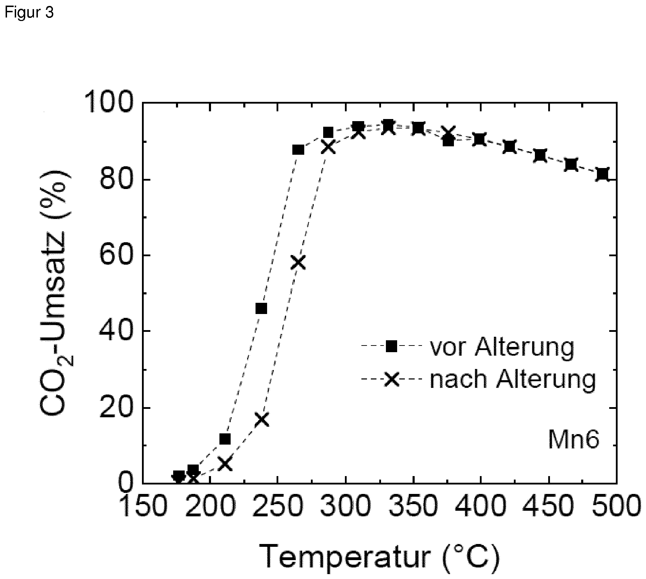

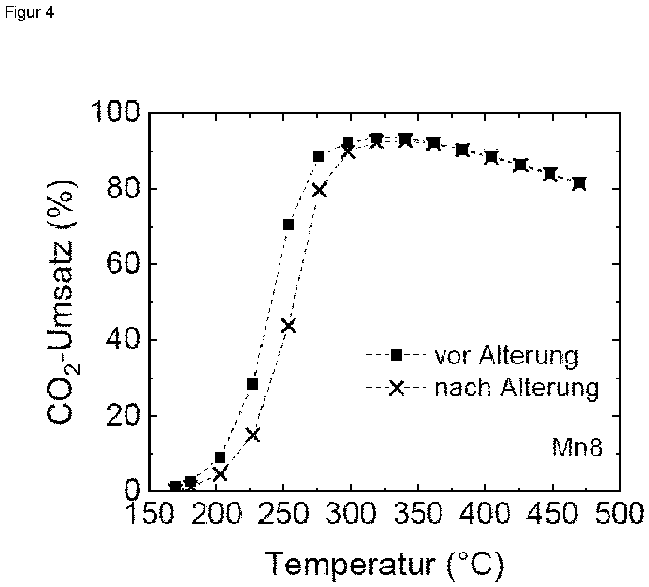

Die Ergebnisse in den Figuren 1 bis 4 zeigen, dass die Promotierung eines Ni/AlO$_x$-Katalysators mit Mangan in einer signifikanten Erhöhung der Katalysatoraktivität resultiert.

**Beispiele**

Tabelle 2: In der Kopräzipitation eingesetzte einmolare Metallsalz-Lösungen

| Bsp.-Nr. | Katalysator | $V_{Ni(NO3)2}$ [ml] | $V_{Al(NO3)3}$ [ml] | $V_{Fe(NO3)3}$ [ml] | $V_{Mn(NO3)2}$ [ml] |
|---|---|---|---|---|---|
| Vergleichsbeispiele | | | | | |
| 1 | Ni | 60,0 | 60,0 | | |
| 2 | Fe2 | 59,0 | 59,0 | 2,0 | |
| 3 | Fe4 | 57,0 | 57,0 | 6,0 | |
| 4 | Fe7 | 55,0 | 55,0 | 10,0 | |
| 5 | Fe10 | 52,5 | 52,5 | 15,0 | |
| 6 | Mn1 | 59 | 59 | - | 2 |
| 7 | Mn4 | 57 | 57 | - | 6 |
| 8 | Mn11 | 51 | 51 | - | 18 |
| 9 | Fe4Mn4 | 54,29 | 54,29 | 5,71 | 5,71 |
| 10 | Fe4Mn1 | 56,10 | 56,10 | 5,90 | 1,90 |

(fortgesetzt)

| Bsp.-Nr. | Katalysator | $V_{Ni(NO3)2}$ [ml] | $V_{Al(NO3)3}$ [ml] | $V_{Fe(NO3)3}$ [ml] | $V_{Mn(NO3)2}$ [ml] |
|---|---|---|---|---|---|
| Vergleichsbeispiele | | | | | |
| 11 | Fe5Mn4 | 53,38 | 53,38 | 7,63 | 5,62 |
| 12 | Fe5Mn1 | 55,13 | 55,13 | 7,88 | 1,87 |
| 13 | Fe6Mn4 | 52,47 | 52,47 | 9,54 | 5,52 |
| 14 | Fe7Mn1 | 54,16 | 54,16 | 9,85 | 1,84 |
| Beispiele | | | | | |
| 1 | Mn6 | 55 | 55 | - | 10 |
| 2 | Mn8 | 52,5 | 52,5 | - | 15 |
| 3 | Fe3Mn6 | 52,47 | 52,47 | 5,52 | 9,54 |
| 4 | Fe5Mn6 | 51,62 | 51,62 | 7,37 | 9,39 |
| 5 | Fe6Mn6 | 50,77 | 50,77 | 9,23 | 9,23 |

Tabelle 3: Zusammensetzung der kalzinierten Katalysatoren

| Beispiel | Katalysator | Elementgehalte [%] [Gew.-%] | | | | Atomare Verhältnisse | | |
|---|---|---|---|---|---|---|---|---|
| | | Ni | Mn | Fe | Al | Ni/Al | Ni/Mn | Ni/Fe |
| Vergleichsbeispiele | | | | | | | | |
| 1 | Ni | 44,3 | - | - | 19,8 | 1,03 | - | - |
| 2 | Fe2 | 40,0 | - | 1,7 | 19,4 | 0,95 | - | 22,0 |
| 3 | Fe4 | 39,7 | - | 4,3 | 19,4 | 0,94 | - | 8,8 |
| 4 | Fe7 | 39,6 | - | 6,9 | 17,3 | 1,05 | - | 5,4 |
| 5 | Fe10 | 36,1 | - | 10,1 | 17,9 | 0,93 | - | 3,4 |
| 6 | Mn1 | 38,7 | 1,3 | - | 18,0 | 0,99 | 27,3 | - |
| 7 | Mn4 | 38,9 | 3,7 | - | 18,2 | 0,98 | 9,8 | - |
| 8 | Mn11 | 34,0 | 10,8 | | 16,2 | 0,97 | 3,0 | - |
| 9 | Fe4Mn4 | 39,9 | 17,8 | 4,2 | 4,0 | 1,03 | 9.0 | 9.5 |
| 10 | Fe4Mn1 | 39,5 | 17,7 | 1,3 | 3,9 | 1,03 | 28,9 | 9,7 |
| 11 | Fe5Mn4 | 38,1 | 17,3 | 3,8 | 5,3 | 1,01 | 9,4 | 6,8 |
| 12 | Fe5Mn1 | 38,7 | 17,4 | 1,2 | 5,3 | 1,02 | 29,4 | 6,9 |
| 13 | Fe6Mn4 | 36,9 | 18,2 | 3,5 | 6,2 | 0,93 | 9,8 | 5,6 |
| 14 | Fe7Mn1 | 37,2 | 16,0 | 1,3 | 6,5 | 1,07 | 26,5 | 5,4 |
| Beispiele | | | | | | | | |
| 1 | Mn6 | 36,3 | 6,1 | - | 15,5 | 1,08 | 5,6 | - |
| 2 | Mn8 | 31,9 | 8,3 | - | 15,2 | 0,97 | 3,6 | - |
| 3 | Fe3Mn6 | 35,6 | 16,0 | 6,0 | 3,4 | 1,02 | 6,0 | 10,0 |
| 4 | Fe5Mn6 | 36,3 | 16,6 | 6,1 | 4,8 | 1,01 | 5,5 | 7,2 |
| 5 | Fe6Mn6 | 34,1 | 16,8 | 5,8 | 5,9 | 0,93 | 5,5 | 5,5 |

Tabelle 4: Charakterisierungsdaten der Katalysatoren

| Beispiel | Katalysator | $S_{BET}^a$ $[m^2 g_{kat}^{-1}]$ | $S_{Met}^a$ $[m^2 g_{kar}^{-1}]$ | $U(CO_2)^a$ $[\mu mol\ g_{kat}^{-1}]$ |
|---|---|---|---|---|
| Vergleichsbeispiele | | | | |
| 1 | Ni | 209 | 21,1 | 172 |
| 2 | Fe2 | 227 | 19,8 | 199 |
| 3 | Fe4 | 244 | 18,3 | 198 |
| 4 | Fe7 | 216 | 11,4 | 196 |
| 5 | Fe10 | 250 | 9,3 | 188 |
| 6 | Mn1 | 211 | 19,2 | 197 |
| 7 | Mn4 | 223 | 20,1 | 215 |
| 8 | Mn11 | 213 | 10,8 | 242 |
| 9 | Fe4Mn4 | 275 | 12,9 | 345 |
| 10 | Fe4Mn1 | 241 | 16,1 | 204 |
| 11 | Fe5Mn4 | 238 | 17,8 | 298 |
| 12 | Fe5Mn1 | 251 | 17,6 | 269 |
| 13 | Fe6Mn4 | 262 | 7,6 | 276 |
| 14 | Fe7Mn1 | 237 | 11,2 | 223 |
| Beispiele | | | | |
| 1 | Mn6 | 231 | 20,0 | 244 |
| 2 | Mn8 | 214 | 17,6 | 240 |
| 3 | Fe3Mn6 | 249 | 11,9 | 277 |
| 4 | Fe5Mn6 | 268 | 15,8 | 327 |
| 5 | Fe6Mn6 | 239 | 5,5 | 322 |
| $^a$ normiert auf Masse des kalzinierten Katalysators | | | | |

Tabelle 5: Ergebnisse der katalytischen Testreaktion

| Beispiel-Nr. | Katalysator | $T_{75,1}$ [°C] | $T_{75,2}$ [°C] | $T_{75,2}$ / $T_{75,1}$ | Normierte Aktivität | Normierte Stabilität |
|---|---|---|---|---|---|---|
| Vergleichsbeispiele | | | | | | |
| 1 | Ni | 289,41 | 314,11 | 1,085 | 1,000 | 1,000 |
| 2 | Fe2 | 279,29 | 296,86 | 1,063 | 1,036 | 1,021 |
| 3 | Fe4 | 275,05 | 293,37 | 1,067 | 1,052 | 1,018 |
| 4 | Fe7 | 276,35 | 286,84 | 1,038 | 1,047 | 1,046 |
| 5 | Fe10 | 290,54 | 319,21 | 1,099 | 0,996 | 0,988 |
| 6 | Mn1 | 282,04 | 300,91 | 1,067 | 1,026 | 1,017 |
| 7 | Mn4 | 274,57 | 293,36 | 1,068 | 1,054 | 1,016 |
| 8 | Mn11 | 269,95 | 289,67 | 1,073 | 1,072 | 1,011 |
| 9 | Fe4Mn4 | 266,34 | 280,27 | 1,052 | 1,087 | 1,031 |
| 10 | Fe4Mn1 | 274,18 | 286,01 | 1,043 | 1,056 | 1,040 |

(fortgesetzt)

| Beispiel-Nr. | Katalysator | $T_{75,1}$ [°C] | $T_{75,2}$ [°C] | $T_{75,2}$ / $T_{75,1}$ | Normierte Aktivität | Normierte Stabilität |
|---|---|---|---|---|---|---|
| Vergleichsbeispiele | | | | | | |
| 11 | Fe5Mn4 | 264,02 | 275,09 | 1,042 | 1,096 | 1,042 |
| 12 | Fe5Mn1 | 262,62 | 271,83 | 1,035 | 1,102 | 1,049 |
| 13 | Fe6Mn4 | 270,01 | 278,48 | 1,031 | 1,072 | 1,052 |
| 14 | Fe7Mn1 | 269,11 | 280,15 | 1,041 | 1,075 | 1,043 |
| Beispiele | | | | | | |
| 1 | Mn6 | 256,71 | 275,50 | 1,073 | 1,127 | 1,011 |
| 2 | Mn8 | 259,31 | 272,67 | 1,052 | 1,116 | 1,032 |
| 3 | Fe3Mn6 | 253,42 | 278,06 | 1,097 | 1,142 | 0,989 |
| 4 | Fe5Mn6 | 258,16 | 281,24 | 1,089 | 1,121 | 0,996 |
| 5 | Fe6Mn6 | 266,56 | 290,13 | 1,088 | 1,086 | 0,997 |

**Patentansprüche**

1. Katalysator zur Methanisierung von Kohlenmonoxid und/oder Kohlendioxid, umfassend Aluminiumoxid, eine Ni-Aktivmasse sowie Mn, **dadurch gekennzeichnet, dass** das molare Ni/Mn-Verhältnis im Katalysator 4,0 bis 6,5 beträgt und das atomare Al/Ni-Verhältnis zwischen 0,5 und 1,5 ist.

2. Katalysator nach Anspruch 2, **dadurch gekennzeichnet, dass** die Ni-Aktivmasse Kristallite mit einem Durchmesser unter 20 nm, bevorzugt unter 10 nm, aufweist.

3. Katalysator nach einem der vorhergehenden Ansprüche, **gekennzeichnet durch** eine $CO_2$-Aufnahmekapazität bei 35°C von größer 200 $\mu$mol/g, vorzugsweise 200 bis 300 $\mu$mol/g.

4. Verwendung eines Katalysators nach einem der vorhergehenden Ansprüche, zur Methanisierung von Kohlenmonoxid und/oder Kohlendioxid mit gasförmigen Wasserstoff.

5. Verfahren zur Herstellung eines Katalysators nach Anspruch 1, umfassend die Schritte:

   a) Kopräzipitation aus einer Lösung, die Al, Ni und Mn in gelöster Form enthält, um einen Niederschlag zu erhalten,
   b) Isolieren des Niederschlags aus Schritt a),
   c) Trocknen des isolierten Niederschlags aus Schritt b) und
   d) Kalzinieren des getrockneten Niederschlags aus Schritt c).

6. Verfahren nach Anspruch 5, **dadurch gekennzeichnet, dass** die Lösung aus Schritt a) eine wässrige Lösung ist.

7. Verfahren nach Anspruch 5 oder 6, **dadurch gekennzeichnet, dass** der Niederschlag in der Lösung für mindestens 30 Minuten gealtert wird.

8. Verfahren nach Anspruch 5 bis 7, **dadurch gekennzeichnet, dass** der isolierte Niederschlag aus Schritt b) gewaschen wird.

9. Verfahren nach Anspruch 5 bis 8, **dadurch gekennzeichnet, dass** die Al, Ni und Mn in gelöster Form, als ionische Verbindungen in der Lösung vorliegen und diese ionischen Verbindungen dasselbe Anion haben.

10. Verfahren nach Anspruch 9, **dadurch gekennzeichnet, dass** das Anion Nitrat, Sulfat, ein Halogenid, Chlorid, oder Acetat ist.

**11.** Verfahren nach einem der Ansprüche 5 bis 10, **dadurch gekennzeichnet, dass** Mn in der Lösung aus Schritt a) in der Oxidationsstufe II oder III vorliegt.

**12.** Verfahren nach Anspruch 11, **dadurch gekennzeichnet, dass** das Kalzinieren des getrockneten Niederschlags bei einer Temperatur von 300 bis 600 °C an Luft erfolgt.

**13.** Verfahren zur Methanisierung von Kohlendioxid und/oder Kohlenmonoxid bei dem ein Gas enthaltend Kohlendioxid und/oder Kohlenmonoxid mit einem Katalysator nach Anspruch 1 in Kontakt gebracht wird.

**14.** Verfahren nach Anspruch 13, bei dem das Gas mit dem Katalysator bei einer Temperatur oberhalb von 200 °C in Kontakt gebracht wird.

**Claims**

**1.** Catalyst for the methanation of carbon monoxide and/or carbon dioxide, comprising aluminium oxide, a Ni active mass and Mn, **characterized in that** the Ni/Mn molar ratio in the catalyst is 4.0 to 6.5 and the Al/Ni atomic ratio is between 0.5 and 1.5.

**2.** Catalyst according to Claim 2, **characterized in that** the Ni active mass contains crystallites with a diameter below 20 mm, preferably below 10 nm.

**3.** Catalyst according to any of the preceding claims, **characterized by** a $CO_2$ uptake capacity at 35°C of greater than 200 $\mu$mol/g, preferably 200 to 300 $\mu$mol/g.

**4.** Use of a catalyst according to any of the preceding claims for the methanation of carbon monoxide and/or carbon dioxide with gaseous hydrogen.

**5.** Method for the preparation of a catalyst according to Claim 1, comprising the steps of:

a) co-precipitating from a solution containing Al, Ni and Mn in dissolved form to obtain a precipitate,
b) isolating the precipitate from step a),
c) drying the isolated precipitate from step b) and
d) calcining the dried precipitate from step c).

**6.** Method according to Claim 5, **characterized in that** the solution from step a) is an aqueous solution.

**7.** Method according to Claim 5 or 6, **characterized in that** the precipitate in the solution is aged for at least 30 minutes.

**8.** Method according to any of Claims 5 to 7, **characterized in that** the isolated precipitate from step b) is washed.

**9.** Method according to Claim 5 to 8, **characterized in that** the Al, Ni and Mn are present in the solution in dissolved form, as ionic compounds, and these ionic compounds have the same anion.

**10.** Method according to Claim 9, **characterized in that** the anion is nitrate, sulfate, a halide, chloride or acetate.

**11.** Method according to any of Claims 5 to 10, **characterized in that** Mn in the solution from step a) is in oxidation state II or III.

**12.** Method according to Claim 11, **characterized in that** the dried precipitate is calcined at a temperature of 300 to 600°C in air.

**13.** Method for the methanation of carbon dioxide and/or carbon monoxide in which a gas containing carbon dioxide and/or carbon monoxide is brought into contact with a catalyst according to Claim 1.

**14.** Method according to Claim 13, in which the gas is brought into contact with the catalyst at a temperature above 200°C.

**Revendications**

1. Catalyseur pour la méthanation de monoxyde de carbone et/ou de dioxyde de carbone, comprenant de l'oxyde d'aluminium, une masse active de Ni ainsi que Mn, **caractérisé en ce que** le rapport molaire Ni/Mn dans le catalyseur est de 4,0 à 6,5 et le rapport atomique Al/Ni est compris entre 0,5 et 1,5.

2. Catalyseur selon la revendication 2, **caractérisé en ce que** la masse active de Ni présente des cristallites dotés d'un diamètre inférieur à 20 nm, préférablement inférieur à 10 nm.

3. Catalyseur selon l'une quelconque des revendications précédentes, **caractérisé par** une capacité d'absorption de $CO_2$ à 35 °C supérieure à 200 $\mu$mole/g, de préférence de 200 à 300 $\mu$mole/g.

4. Utilisation d'un catalyseur selon l'une quelconque des revendications précédentes, pour la méthanation de monoxyde de carbone et/ou de dioxyde de carbone avec de l'hydrogène gazeux.

5. Procédé pour la préparation d'un catalyseur selon la revendication 1, comprenant les étapes :

   a) coprécipitation à partir d'une solution, qui contient Al, Ni et Mn sous forme dissoute, pour obtenir un précipité,
   b) isolement du précipité de l'étape a),
   c) séchage du précipité isolé de l'étape b) et
   d) calcination du précipité séché de l'étape c).

6. Procédé selon la revendication 5, **caractérisé en ce que** la solution de l'étape a) est une solution aqueuse.

7. Procédé selon la revendication 5 ou 6, **caractérisé en ce que** le précipité est vieilli dans la solution pendant au moins 30 minutes.

8. Procédé selon les revendications 5 à 7, **caractérisé en ce que** le précipité isolé de l'étape b) est lavé.

9. Procédé selon les revendications 5 à 8, **caractérisé en ce que** Al, Ni et Mn sous forme dissoute sont présents dans la solution en tant que composés ioniques et ces composés ioniques possèdent le même anion.

10. Procédé selon la revendication 9, **caractérisé en ce que** l'anion est nitrate, sulfate, un halogénure, chlorure ou acétate.

11. Procédé selon l'une quelconque des revendications 5 à 10, **caractérisé en ce que** Mn est présent dans la solution de l'étape a) dans l'état d'oxydation II ou III.

12. Procédé selon la revendication 11, **caractérisé en ce que** la calcination du précipité séché est réalisée à une température de 300 à 600 °C à l'air.

13. Procédé pour la méthanation de dioxyde de carbone et/ou de monoxyde de carbone dans lequel un gaz contenant du dioxyde de carbone et/ou du monoxyde de carbone est mis en contact avec un catalyseur selon la revendication 1.

14. Procédé selon la revendication 13, dans lequel le gaz est mis en contact avec le catalyseur à une température supérieure à 200 °C.

Figur 1

Figur 2

Figur 3

Figur 4

Figur 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 104043454 A
- CN 103480375 A
- CN 102091629 A
- CN 104888783 A
- WO 2008110331 A1
- CN 103464163 A
- CN 103752315 A
- CN 102600860 A
- CN 102553610 A
- CN 102527405 A
- CN 102513124 A
- CN 102463120 A
- CN 102463119 A
- CN 103706366
- CN 106858268 A
- CN 104028270
- CN 101745401

**In der Beschreibung aufgeführte Nicht-Patentliteratur**

- **ZHAO et al.** *Journal of Natural Gas Chemistry,* 2012, vol. 21 (2), 170-177
- **WAN AZELEE WAN ABU BAKAR et al.** *Catalysis Letters,* 2008, vol. 128 (1-2), 127-136
- **ZHAO KECHAO et al.** *Frontiers of Chemical Science and Engineering,* 2016, vol. 10 (2), 273-280
- **GAO et al.** *Journal of molecular catalysis (China),* Februar 2011, vol. 25 (1